# EUROPÄISCHE PATENTSCHRIFT

(11) **EP 1 922 996 B1**
(45) Veröffentlichungstag und Bekanntmachung des Hinweises auf die Patenterteilung: **09.03.2016**
(21) Anmeldenummer: 07020606.5
(22) Anmeldetag: 22.10.2007
(51) Int. Cl.: A61B 5/103

(54) **Vorrichtung zur Messung der Innenrotation des Hüftgelenkes**
Device for measuring the inner rotation of a hip joint
Dispositif de mesure de la rotation interne de l'articulation de la hanche

(30) Priorität: 23.10.2006 CH 16902006
(43) Veröffentlichungstag der Anmeldung: 21.05.2008
(73) Patentinhaber: Leunig, Michael, 8703 Erlenbach (CH)
(72) Erfinder: Leunig, Michael, 8703 Erlenbach (CH)
(74) Vertreter: Lusuardi, Werther

(56) Entgegenhaltungen:
- EP-A- 0 275 956
- US-A1- 2004 082 437
- CROFT P R ET AL: "Interobserver reliability in measuring flexion, internal rotation, and external rotation of the hip using a plurimeter." ANNALS OF THE RHEUMATIC DISEASES MAY 1996, Bd. 55, Nr. 5, Mai 1996 (1996-05), Seiten 320-323, XP002474550 ISSN: 0003-4967

## Beschreibung

Die Erfindung bezieht sich auf eine Vorrichtung zur Messung der Innenrotation des Hüftgelenkes gemäss dem Oberbegriff des Patentanspruchs 1.

Aus der japanischen Patentpublikation JP2004097489 TABUCHI ist ein medizinischer Untersuchungstisch in Form eines Stuhles bekannt, welcher in seinen Positionen sehr genau eingestellt werden kann, das heisst die Position der Rückenlehne, des Sitzes oder aber der Beinhalterungen. Es wird jedoch keine Messung der Innendrehung des Hüftgelenkes offenbart.

Aus dem russischen Patent Nr. 2 170 542 CHERNOVA ist ein Verfahren und eine Vorrichtung zur Bestimmung der Position des Beckens bekannt. Dabei wird die Deflexion und Innenrotation der Femora in Rückenlage gemessen. Die Innendrehung wird so durchgeführt, dass die Schienbeine durch Aufbringen einer geeignet variierenden Kraft nach aussen geführt werden. Neben der Innenrotation wird bei CHERNOVA auch die Veränderung der Beckenposition in Abhängigkeit der Femurrotation gemessen. Ferner gestattet die Rückenlage nur eine geringe Fixierung des Beckens durch das Körpergewicht.

Aus dem US Patent 6,773,376 DVIR ist ein Verfahren zur Ermittlung der Innenrotation des Hüftgelenkes bekannt, bei welchem Winkelmessungen isokinetisch mittels eines linearen Dynamometers durchgeführt werden. In FIG 5B ist die Position offenbart, in welcher die Innenrotation des Hüftgelenkes gemessen wird. Nachteilig bei dieser bekannten Methode ist der Umstand, dass hier eine isokinetische Messung, d.h. bei einer konstanten Geschwindigkeit der Bewegung durchgeführt wird. Die Intention dieses Verfahrens ist somit rein auf der muskulären Seite und lässt keine Quantifizierung des Effektes knöcherner Alterationen zu.

Keines der oben erwähnten Dokumente beschreibt eine Messung der Innenrotation des Hüftgelenkes mittels einer konstanten Kraft.

Der Erfindung liegt daher die Aufgabe zugrunde, eine Vorrichtung zur Messung der Innenrotation des Hüftgelenkes, insbesondere des erreichbaren Winkels bei einer durch eine konstante Kraft erzielten Rotation des Oberschenkels um dessen Längsachse zu schaffen.

Die erhaltenen Messresultate stellen einen Indikator für allfällige Schäden im Gelenk dar. Bei reduzierter Innenrotation des Hüftgelenkes kann frühzeitig ein Schaden am Hüftgelenk auftreten.

Die Erfindung löst die gestellte Aufgabe mit einer Vorrichtung, welche die Merkmale des Anspruchs 1 aufweist.

Die durch die Erfindung erreichten Vorteile sind im wesentlichen darin zu sehen, dass:
a) die Patienten nicht auf dem Rücken liegen, sondern sitzen, damit wird das Becken durch das Körpergewicht gut fixiert; und
b) die passive, nicht aktive Beweglichkeit (Muskelkraft) untersucht werden kann, so dass endphasisch quantifiziert werden kann, inwieweit ohne Muskelgegenhalt die Innenrotation möglich ist. Dazu wird auch eine definierte Kraft eingesetzt, so dass sich mittels Erfassung des femoroacatabularen Zusammenstossens der Effekt knöcherner Alterationen quantifizieren lässt.

Ferner, konnte beim Einsatz der erfindungsgemässen Vorrichtung nachgewiesen werden, dass
1) die Inter- und Intraraterreliabilität der erfindungsgemässen Messungen im Vergleich zur herkömmlichen Messung (die nicht mit einer definierten Sitzposition und Kraftaufwendung durchgeführt wird) hervorragend ist; und
2) eine verminderte Innenrotation mit einer Gelenksschädigung vergesellschaftet ist. Pro 10° weniger Innenrotation steigt die im MRI nachgewiesene Degeneration des Labrum.

In verschiedenen Ausführungsformen der erfindungsgemässen Vorrichtung können als Mittel zum Erzeugen einer Kraft K beispielsweise die folgenden Vorrichtungen eingesetzt werden, wobei die Kraft K am Unterschenkel vorzugsweise derart eingeleitet wird, dass ihre Wirkungsachse im wesentlichen parallel zur Tischfläche des Untersuchungstisches verläuft:
1) An einem Seilzug befestigbare Gewichtskörper, beispielsweise analog zu den heute üblichen Geräten für Krafttraining. Solche Geräte gestatten die Erzeugung einer linearen konstanten Zugkraft, welche über die beim Fussgelenk am Unterschenkel befestigte Fixationseinrichtung auf den Unterschenkel übertragen wird. Der Unterschenkel wird durch die auf ihn ausgeübte Zugkraft um die Längsachse des zugehörigen Oberschenkels gedreht, so dass das Fussgelenk von der Sagittalebene weggeführt wird und der Oberschenkel um seine Längsachse um den Winkel α gedreht wird.
2) Druck- oder Zugfeder, z.B. eine Schraubenfeder, deren Federweg durch eine geeignete Begrenzungseinrichtung auf eine Länge s begrenzt wird, so dass beim Erreichen der Länge s eine konstante Kraft K mit dem gewünschten Betrag Z über die beim Fussgelenk am Unterschenkel befestigte Fixationseinrichtung auf den Unterschenkel übertragen wird.
3) Druckluftzylinder mit einem Druckbegrenzungsventil zum Einstellen der maximal auszuübenden Zug- oder Druckkraft. Zur Übertragung der Zugkraft ist das vordere Ende der Kolbenstange mit der Fixationseinrichtung verbunden, so dass beim Ansprechen des Druckbegrenzungsventils eine Kraft K mit dem gewünschten Betrag Z über die beim Fussgelenk am Unterschenkel befestigte Fixationseinrichtung auf den Unterschenkel übertragen wird.
4) Elektromotor, welcher vorzugsweise ein Getriebe, eine Drehmomentbegrenzung und eine Seilwinde umfasst, wobei das eine Ende des Seilzuges an der Fixationseinrichtung befestigt ist, so dass mittels der Drehmomentbegrenzung eine konstante Kraft K mit dem gewünschten Betrag Z über die beim Fussgelenk am Unterschenkel befestigte Fixationseinrichtung auf den Unterschenkel übertragbar ist.
5) Elektrischer Linearmotor mit einer regulierbaren elektrischen oder elektronischen Kraftbegrenzung, so dass mittels der Kraftbegrenzung eine konstante Kraft K mit dem gewünschten Betrag Z über die beim Fussgelenk am Unterschenkel befestigte Fixationseinrichtung auf den Unterschenkel übertragen wird.

In einer anderen Ausführungsform umfasst die Vorrichtung zusätzlich eine Zeiterfassungsvorrichtung, welche beispielsweise aus einer vom Operateur ablesbaren Uhr oder eine elektronische Zeitablaufsteuerung in den Mitteln zum Erzeugen einer Kraft K umfasst. Damit ist erreichbar, dass die Kraft K mindestens während einer definierten Mindestzeitspanne ΔT > 0 am Unterschenkel angreift. Vorzugsweise erfolgt die Messung nach Erreichen des Äquilibriums der Innendrehung. Eine möglichst lange Zeitspanne für die Krafteinwirkung ist daher für das Erreichen eines optimalen Resultates wünschbar.

In einer weiteren Ausführungsform beträgt die Mindestzeitspanne ΔT mindestens 25 Sekunden.

In einer weiteren Ausführungsform umfassen die Mittel zum Erzeugen einer Kraft K ein Dynamometer.

In wiederum einer weiteren Ausführungsform ist der Sockel auf einem medizinischen Untersuchungstisch lösbar befestigbar.

In einer anderen Ausführungsform sind die Mittel zum Erzeugen einer Kraft K an einem medizinischen Untersuchungstisch lösbar befestigbar sind.

In einer weiteren Ausführungsform ist die Rückenlehne relativ zur Sitzfläche rotierbar und fixierbar, so dass die Anlagefläche mit der Sitzfläche einen Winkel β zwischen 85° und 95° einschliesst. Damit ist der Vorteil erreichbar, dass das Becken der zu untersuchenden Person durch das Körpergewicht gut fixiert wird.

In einer anderen Ausführungsform weist die mindestens eine Fixationseinrichtung orthogonal zur Sitzfläche gemessen einen wählbaren Abstand X > 0 zur Sitzfläche auf. Der Abstand X wird derart eingestellt, dass die Kraft K in einem bestimmten, parallel zur Längsachse des Unterschenkels gemessenen Abstand A vom Zentrum des Knies am Unterschenkel angreift. Der Abstand A sollte mindestens die Unterschenkellänge betragen. Vorteilhaft ist eine möglichst gmsse Länge, weiche jedoch durch die Höhe des Untersuchungstisches begrenzt wird.

In wiederum einer anderen Ausführungsform schliesst die Wirkungslinie der Kraft K mit der in der Sagittalebene liegenden Normalen der Sitzfläche einen Winkel Gamma zwischen 80° und 100° ein. Damit ist der Vorteil erreichbar, dass durch einen Kraftangriffswinkel nahe bei 90° keine beinaufwärts wirkenden Reibungskräfte auf den Unterschenkel übertragen werden.

In wiederum einer weiteren Ausführungsform beträgt der Betrag Z der Kraft K mindestens 40 Newton.

In einer weiteren Ausführungsform umfassen die Mittel zum Messen eines Winkels α zwischen der Sagittalebene und der Längsachse des Unterschenkels einer zu untersuchenden Person ein Goniometer. Anstelle eines Goniometers kann auch eine Vermessung mittels einer der üblicherweise in der Computer-Assistierten Chirurgie oder bei chirurgischen Navigationsvorrichtungen eingesetzten, opto-elektronischen Positionserfassungsvorrichtungen erfolgen.

### Kurze Beschreibung verschiedener Ausführungsformen des erfindungsgemässen Verfahrens:

Das Verfahren zur Messung der Innenrotation eines Hüftgelenkes, vorzugsweise mit Hilfe der erfindungsgemässen Vorrichtung, umfasst im wesentlichen die Schritte:
a) Positionieren einer zu untersuchenden Person auf der Sitzfläche einer stuhlähnlichen Positionierhilfe in sitzender Stellung;
b) Fixierung beider zu untersuchenden Oberschenkel der Person auf der Sitzfläche bezüglich translativer Bewegungen, so dass eine Rotation der Oberschenkel um ihre Längsachse weiterhin möglich ist;
c) Bewegen der entsprechenden Unterschenkel quer zur Sagittalebene mittels einer an je einem Unterschenkel angreifenden Kraft K eines konstanten Betrages Z, so dass die distalen Enden der Unterschenkel von der Sagittalebene weggeführt werden; und
d) Messen des Winkels α zwischen der Sagittalebene und der Längsachse des Unterschenkels.

Ein Vorteil dieses Verfahrens liegt darin, dass die Messung der Innenrotation symmetrisch, d.h. gleichzeitig an beiden Hüftgelenken einer zu untersuchenden Person gemessen wird, so dass das Becken nicht ausweichen kann.

In einer bevorzugten Ausführungsform greift die Kraft K während einer definierten Mindestzeitspanne ΔT > 0 am Unterschenkel an.

In einer weiteren Ausführungsform beträgt die Mindestzeitspanne ΔT mindestens 25 Sekunden.

Eine bevorzugte Ausführungsform des erfindungsgemässen Verfahrens zeichnet sich dadurch aus, dass eine statische definierte Kraft (5 kg) über 30 Sekunden am Unterschenkel einwirken soll, sodass dieser nach aussen geführt wird und damit eine Innendrehung der Hüfte entsteht. Es werden keine aktiven Messungen ausgeführt (Muskel), sondern rein die passive Beweglichkeit (als standardisierte Screeningmethode für die Innenrotation) untersucht. Neben einem Gewicht als definierte Auslenkkraft, kann auch ein lineares Dynamometer eingesetzt werden.

In wiederum einer weiteren Ausführungsform erfolgt die Einstellung einer an der Positionierhilfe bewegbaren Rückenlehne mit einer zur Anlage an den Rücken der zu untersuchenden Person bestimmten Anlagefläche so, dass die Anlagefläche mit der Sitzfläche einen Winkel β zwischen 85° und 95° einschliesst.

Eine andere Ausführungsform zeichnet sich dadurch aus, dass die Kraft K eine reine Zugkraft ist und in einem bestimmten, parallel zur Längsachse des Unterschenkel gemessenen Abstand A vom Zentrum des Knies am Unterschenkel angreift. Der Abstand A sollte mindestens die Unterschenkellänge betragen. Vorteilhaft ist ein möglichst grosser Abstand A, welcher jedoch durch die Höhe des Untersuchungstisches begrenzt wird.

In einer weiteren Ausführungsform liegt die Wirkungslinie der Kraft K in einer zur Sagittalebene und zur Transversalebene senkrechten Ebene und schliesst mit der Längsachse des Unterschenkels einen Winkel Gamma zwischen 80° und 100° ein. Damit ist der Vorteil erreichbar, dass durch einen Kraftangriffswinkel nahe bei 90° keine beinaufwärts wirkenden Reibungskräfte auf den Unterschenkel übertragen werden.

In wiederum einer anderen Ausführungsform beträgt der Betrag Z der Kraft K mindestens 40 Newton.

In einer weiteren Ausführungsform erfolgt das unter Schritt d) ausgeführte Messen des Winkels α mittels eines Goniometers. Anstelle eines Goniometers kann auch eine Vermessung mittels einer der üblicherweise in der Computer-Assistierten Chirurgie oder bei chirurgischen Navigationsvorrichtungen eingesetzten, opto-elektronischen Positionserfassungsvorrichtungen erfolgen. Weitere Möglichkeiten zur Vermessung des Winkels α sind die Vermessung von Fotodokumentationen oder eine mechanisch digitale Auslenkungsbestimmung.

In einer anderen Ausführungsform wird vor Schritt a) der folgende Schritt ausgeführt: Fixieren der Positionierhilfe auf einem medizinischen Untersuchungstisch.

Die Erfindung wird im folgenden anhand der teilweise schematischen Darstellung eines Ausführungsbeispiels noch näher erläutert.

Es zeigen:

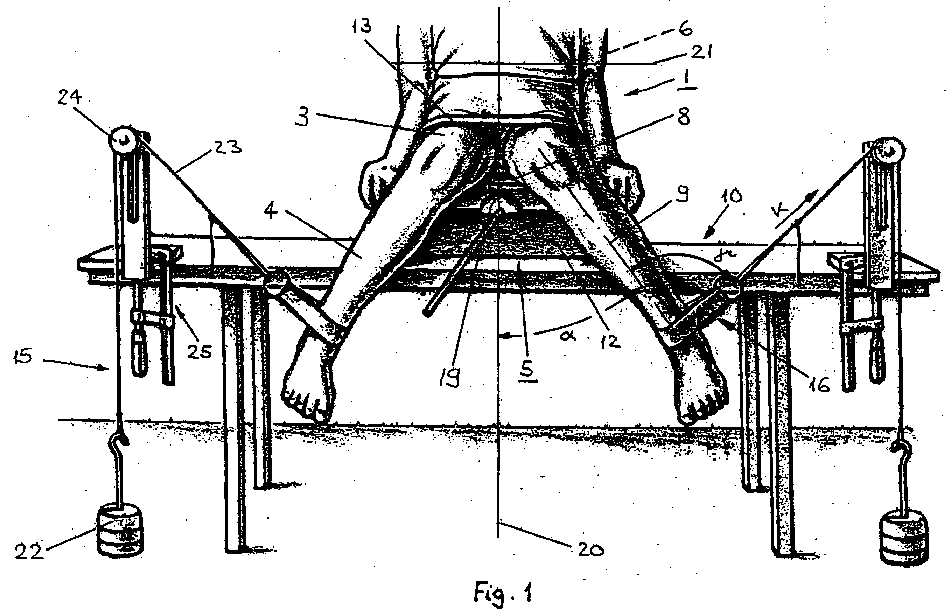

Fig. 1 eine schematische Darstellung einer auf einem medizinischen Untersuchungstisch montierten Ausführungsform der erfindungsgemässen Positionierhilfe.

Die in Fig. 1 dargestellte Positionierhilfe 5 umfasst im wesentlichen einen Sockel 12 mit einer Sitzfläche 8 und einer eine Anlagefläche (nicht sichtbar) aufweisenden Rückenlehne 6, Befestigungsmittel 13 zur Fixierung beider Oberschenkel 3 der zu untersuchenden Person 1 auf der Sitzfläche 8 bezüglich translativer Bewegungen, so dass eine Rotation der Oberschenkel 3 um deren Längsachse (senkrecht zur Zeichenebene) weiterhin möglich ist und Mittel 15 zum-Erzeugen einer Kraft K parallel zu einer Wirkungsachse, welche einseitig mittels Fixationseinrichtungen 16 an den entsprechenden Unterschenkeln 4 lösbar befestigbar sind.

Der Sockel 12 der Positionierhilfe 5 ist beispielsweise mittels üblichen Klemmelementen (nicht gezeichnet) so auf einem medizinischen Untersuchungstisch 10 befestigt, dass die Längsachsen der Oberschenkel 3 der zu untersuchenden Person 1 parallel zur kurzen Seite der Tischfläche des Untersuchungstisches 10 verlaufen und die Unterschenkel 4 über eine der langen Seiten der Tischfläche des Untersuchungstisches 10 hinausragen und frei hängen.

Als Mittel 15 zum Erzeugen einer Kraft K dienen in der hier dargestellten Ausführungsform an einem Seilzug 23 befestigbare Gewichtskörper 22. Der Seilzug 23 umfasst ein Seil und eine Umlenkrolle 24, deren Distanz zum Untersuchungstisch 10 verstellbar ist und welche beispielhaft mit einer Schraubklemme 25 am Untersuchungstisch 10 befestigt ist. An einem Ende des Seiles sind die Gewichtskörper 22 befestigt, während das andere Ende des Seiles mittels Fixationseinrichtungen 16, z.B. mittels Riemen beim Fussgelenk mit dem Unterschenkel 4 verbunden ist. Die hier dargestellte Ausführungsform umfasst symmetrisch zur Sagittalebene 20 je einen Seilzug 23 für das linke und das rechte Bein der zu untersuchenden Person 1.

Der Unterschenkel 4 wird durch die auf ihn ausgeübte Zugkraft um die Längsachse des zugehörigen Oberschenkels gedreht, so dass das Fussgelenk von der Sagittalebene 20 (senkrecht zur Zeichenebene) weggeführt wird und der Oberschenkel 3 um seine Längsachse um den Winkel α gedreht wird.

Ferner ist die relativ zur Sitzfläche 8 rotierbare Rückenlehne 6 so fixiert, dass ihre Anlagefläche (nicht sichtbar) für den Rücken der zu untersuchenden Person 1 mit der Sitzfläche 8 einen Winkel β (nicht gezeichnet) von 90° einschliesst.

Das Messen des Winkels α zwischen der Sagittalebene 20 und der Längsachse 9 des Unterschenkels 4 erfolgt mittels des am Sockel 12 angeordneten Goniometers 19.

## Patentansprüche

1. Vorrichtung zur Messung der Innenrotation des Hüftgelenkes mit einer Positionierhilfe (5) umfassend:
A) einen eine Sitzfläche (8) und eine Rückenlehne (6) mit einer Anlagefläche aufweisenden Sockel (12);
B) Mittel (15) zum Erzeugen einer Kraft K eines konstanten Betrages Z und parallel zu einer Wirkungsachse, welche einseitig mittels einer Fixationseinrichtung (16) am Unterschenkel (4) einer zu untersuchenden Person (1) lösbar befestigbar sind, und mit
C) Mitteln zum Messen eines Winkels α zwischen der Sagittalebene (20) und der Längsachse (9) des entsprechenden Unterschenkels (4)
**dadurch gekennzeichnet, dass**
die Vorrichtung zusätzlich umfasst:
D) Befestigungsmittel (13) zur Fixierung mindestens eines Oberschenkels (3) einer zu untersuchenden Person (1) auf der Sitzfläche (8) bezüglich translativer Bewegungen, so dass eine Rotation des Oberschenkels (3) um dessen Längsachse weiterhin möglich ist; und
E) eine Zeiterfassungsvorrichtung.

2. Vorrichtung nach Anspruch 1, **dadurch gekennzeichnet, dass** die Mittel (15) zum Erzeugen einer Kraft K ein Dynamometer umfassen.

3. Vorrichtung nach Anspruch 1 oder 2, **dadurch gekennzeichnet, dass** der Sockel (12) auf einem medizinischen Untersuchungstisch lösbar befestigbar ist.

4. Vorrichtung nach einem der Ansprüche 1 bis 3, **dadurch gekennzeichnet, dass** die Mittel (15) zum Erzeugen einer Kraft K an einem medizinischen Untersuchungstisch (10) lösbar befestigbar sind.

5. Vorrichtung nach einem der Ansprüche 1 bis 4, **dadurch gekennzeichnet, dass** die Rückenlehne (6) relativ zur Sitzfläche (8) rotierbar ist, so dass die Anlagefläche mit der Sitzfläche (8) einen Winkel β zwischen 85° und 95° einschliesst.

6. Vorrichtung nach einem der Ansprüche 1 bis 5, **dadurch gekennzeichnet, dass** die mindestens eine Fixationseinrichtung (16) orthogonal zur Sitzfläche (8) gemessen einen wählbaren Abstand X > 0 zur Sitzfläche (8) aufweist.

7. Vorrichtung nach einem der Ansprüche 1 bis 6, **dadurch gekennzeichnet, dass** die Wirkungslinie der Kraft K mit der Normalen der Sitzfläche (8) einen Winkel Gamma zwischen 80° und 100° einschliesst.

8. Vorrichtung nach einem der Ansprüche 1 bis 7, **dadurch gekennzeichnet, dass** der Betrag Z der Kraft K mindestens 40 Newton beträgt.

9. Vorrichtung nach einem der Ansprüche 1 bis 8, **dadurch gekennzeichnet, dass** die Mittel (15) zum Erzeugen einer Kraft K mindestens einen Gewichtskörper (22), einen Seilzug (23) und mindestens eine an einem medizinischen Untersuchungstisch lösbar befestigbare Umlenkrolle (24) für den Seilzug (23) umfassen, wobei das erste Ende des Seilzuges (23) mit dem Gewichtskörper (22) verbunden ist und das zweite Ende des Seilzuges (23) an einem Unterschenkel (4) einer zu untersuchenden Person (1) befestigbar ist.

## Claims

1. A device for measuring the internal rotation of a hip joint using a positioning aid (5), comprising:
A) a pedestal (12) including a seating surface (8) and a backrest (6) having a bearing surface;
B) means (15) for generating a force K having a constant magnitude Z and in parallel to an axis of action, which can be unilaterally and detachably fastened by means of a fixation device (16) to the lower thigh (4) of a person (1) to be examined, and
C) means for measuring an angle α between the sagittal plane (20) and the longitudinal axis (9) of the corresponding lower thigh (4),
**characterized in that** the device also comprises:
D) fastening means (13) for fixating at least one upper thigh (3) of a person (1) to be examined to the seating surface (8) with respect to translational movements, so that a rotation of the upper thigh (3) about the longitudinal axis thereof is still possible; and
E) a time recording device.

2. The device according to Claim 1, **characterized in that** the means (15) for generating a force K comprise a dynamometer.

3. The device according to Claim 1 or 2, **characterized in that** the pedestal (12) can be detachably fastened to a medical examination table.

4. The device according to one of Claims 1 through 3, **characterized in that** the means (15) for generating a force K can be detachably fastened to a medical examination table (10).

5. The device according to one of Claims 1 through 4, **characterized in that** the backrest (6) can be rotated relative to the seating surface (8) so that the bearing surface forms an angle ß between 85° and 95° with the seating surface (8).

6. The device according to one of Claims 1 through 5, **characterized in that** the at least one fixation device (16), measured orthogonally to the seating surface (8), has a selectable distance X > 0 from the seating surface (8).

7. The device according to one of Claims 1 through 6, **characterized in that** the line of action of the force K forms an angle gamma between 80° and 100° with the normal of the seating surface (8).

8. The device according to one of Claims 1 through 7, **characterized in that** magnitude Z of the force K is at least 40 Newton.

9. The device according to one of Claims 1 through 8, **characterized in that** the means (15) for generating a force K comprise at least one weight (22), one tackle (23) and at least one deflecting roller (24) for the tackle (23) which can be detachably fastened to a medical examination table, the first end of the tackle (23) being connected to the weight (22) and the second end of the tackle (23) being fastenable to a lower thigh (4) of a person (1) to be examined.

## Revendications

1. Dispositif de mesure de la rotation interne de l'articulation de la hanche avec une aide au positionnement (5), comprenant :
A) un socle (12) présentant une assise (8) et un dossier (6) avec une surface d'appui ;
B) des moyens (15) de production d'une force K d'une valeur Z constante et parallèlement à un axe d'action, qui peuvent être fixés d'un côté de façon détachable au moyen d'un dispositif de fixation (16) sur la jambe (4) d'une personne (1) à examiner, et avec
C) des moyens de mesure d'un angle α entre le plan sagittal (20) et l'axe longitudinal (9) de la jambe (4) correspondante,
**caractérisé en ce que**
le dispositif comprend en plus :
D) des moyens d'immobilisation (13) pour la fixation d'au moins une cuisse (3) d'une personne (1) à examiner sur l'assise (8) par rapport à des mouvements de translation, de telle sorte qu'une rotation de la cuisse (3) demeure possible autour de son axe longitudinal ; et
E) un dispositif d'enregistrement du temps.

2. Dispositif selon la revendication 1, **caractérisé en ce que** les moyens (15) de production d'une force K comprennent un dynamomètre.

3. Dispositif selon la revendication 1 ou 2, **caractérisé en ce que** le socle (12) peut être fixé de façon détachable sur une table d'examen médicale.

4. Dispositif selon l'une des revendications 1 à 3, **caractérisé en ce que** les moyens (15) de production d'une force K peuvent être immobilisés de façon détachable sur une table d'examen médicale (10).

5. Dispositif selon l'une des revendications 1 à 4, **caractérisé en ce que** le dossier (6) peut tourner par rapport à l'assise (8) de telle sorte que la surface d'appui forme avec l'assise (8) un angle β entre 85° et 95°.

6. Dispositif selon l'une des revendications 1 à 5, **caractérisé en ce que** le dispositif de fixation (16) au moins au nombre de un présente, mesuré à angle droit par rapport à l'assise (8), une distance sélectionnable X > 0 par rapport à l'assise (8).

7. Dispositif selon l'une des revendications 1 à 6, **caractérisé en ce que** la ligne d'action de la force K forme avec la normale de l'assise (8) un angle gamma entre 80° et 100°.

8. Dispositif selon l'une des revendications 1 à 7, **caractérisé en ce que** la valeur Z de la force K est d'au moins 40 newtons.

9. Dispositif selon l'une des revendications 1 à 8, **caractérisé en ce que** les moyens (15) de production d'une force K comprennent au moins un poids (22), une commande par câble (23) et au moins une poulie de renvoi (24) destinée à la commande par câble (23) et pouvant être immobilisée de façon détachable sur une table d'examen médicale (10), la première extrémité de la commande par câble (23) étant raccordée au poids (22), et la deuxième extrémité de la commande par câble (23) pouvant être immobilisée sur une jambe (4) d'une personne (1) à examiner.
